# EUROPEAN PATENT SPECIFICATION

(11) **EP 2 777 747 B1**
(45) Date of publication and mention of the grant of the patent: **10.05.2017**
(21) Application number: 13290062.2
(22) Date of filing: 15.03.2013
(51) Int. Cl.: A61M 25/06, A61M 25/00, A61M 25/09

(54) **An access sheath**
Zugangshülle
Gaine d'accès

(43) Date of publication of application: 17.09.2014
(73) Proprietor: Coloplast A/S, 3050 Humlebæk (DK)
(72) Inventor: Callede, David, 24200 Sarlat la Caneda (FR); Lafitte, Mathieu, 24200 Sainte Nathalene (FR); Pascal, Laurent, 24200 Sarlat La Caneda (FR)

(56) References cited:
- WO-A1-97/29680
- WO-A1-2009/127216
- FR-A1- 2 757 406
- US-A1- 2004 143 286
- US-A1- 2006 030 864

## Description

The invention relates to access sheaths through which surgeons can make an intervention with one or more tools within the human body. More particularly, the invention relates to an assembly of parts for an endourological procedure and to a method for carrying out an endourological procedure using the assembly of parts.

### Background

If a surgeon needs to access a kidney in a patient, e.g. in order to remove a kidney stone, and if a direct surgical intervention has been dismissed, the natural route of entry is as follows: going through the urethra into the bladder, passing the bladder, going beyond the ureteral meatus and then going through the ureter to reach the kidney.

The positioning of an access sheath has traditionally been performed by a quite restrictive method explained below.

Following the insertion of an endoscope into the bladder to locate the ureteral meatus, a first radiopaque guidewire is driven up to the bladder and then, with the aid of the endoscope targeting the ureteral meatus for introduction of the first guidewire into the ureter.

After the first guidewire has been set up, a radiopaque double channel ureteral probe is engaged by one of its both channels onto the first guidewire and it is driven up to the ureter. Through the other channel of the ureteral probe, a second guidewire is introduced until it too reaches the ureter. Then the ureteral probe is removed to leave the first and the second guidewires in the patient, one guide now functioning as a working guidewire and the other one functioning as a security guidewire. The security guidewire is then fixed to, and held in place on, the patient. During these first steps the radiopaque components have been visualized to check their positions by means of an X-ray cannon connected to a monitor.

The access sheath to be positioned sits, or is threaded onto, an introducer element projecting proximally beyond the access sheath, the introducer comprising a channel. The access sheath on the introducer is then guided onto the working guidewire through the channel and the sheath is then driven up to a position between the bladder and the kidney, however relatively nearer to the bladder. The introducer element and the working guidewire are then removed to leave behind in place only the access sheath, and near it, the security guidewire that can be used in case of unforeseen difficulties.

The course of all these steps of positioning the access sheath highlights the fundamental problem behind the present invention: reducing the number of positioning steps in order to save time and reduce the risks for the patient involved with the procedure while limiting the number of necessary components (including the working and security guidewires, the ureteral probe for positioning the security guidewire and the dilator element).

WO2009/127216A1 relates to a catheter for positioning an access sheath and a security guide next to the sheath for an intervention in a difficult-to-access area within the body, comprising, along the catheter, at least one security channel for positioning a guide, characterised by the fact that the security channel is a channel extending between an outer hole and an inner hole, the wall of the catheter extending between both holes of the security channel being arranged to open under the action of a peel strength directed from inside to outside the channel.

US2007/0250001A1 discloses a guidewire separator device for resolving guidewire entanglement and for introducing guidewires into branch vessels when performing angioplasty or stenting of a bifurcated artery. The device has first and second lumens and may optionally include one or more longitudinal slits to externalize the guidewires from the lumens during withdrawal of the device.

FR2757406 discloses a tubular guide sleeve for inserting a catheter into a vein. The tubular guide sleeve is disclosed having a preformed lengthwise line of reduced strength which allows the tubular guide sleeve to be split lengthwise and removed after the insertion of a catheter through a lateral inlet situated at one end of the line.

WO 97/29680 discloses a surgical access device comprising an introducer with a main endoscope channel and an auxiliary or secondary instrument channel. The introducer exhibits an extremely narrow cross-sectional profile in order to minimize the size of the portal or other entry point necessary to gain access into the patient's body.

### Summary of the Invention

The present invention relates to access sheaths to aid surgeons in performing certain endoscope-assisted surgical procedures, i.e. procedures being performed inside the body of a patient by means of a scope being connected to a monitor allowing the surgeon to see the site of interaction. The access sheaths may be especially useful in an endourological procedure however the present application is also intended to embrace the use of the invention in other procedures where suitable. Particularly, the invention relates to an assembly of parts facilitating the use of a single guidewire, contrary to hitherto known procedures requiring both a working guidewire and a security guidewire thus meaning that less insertions and retractions of surgical devices to and from the patient are needed.

### Brief Description of the Drawing

Figure 1 is a schematic perspective view of an assembly of parts according to some embodiments of the invention,
Figure 2 is a schematic perspective view of an elongate introducer of the assembly of parts according to some embodiments of the invention,
Figure 2a is a cross-sectional view of embodiments of the elongate introducer without a security channel,
Figure 2b is another cross-sectional view of alternative embodiments wherein the elongate introducer includes a security channel,
Figure 3a is a schematic perspective view of embodiments of a tubular sheath of an assembly of parts according to the invention comprising a second lumen defined by a sheet material,
Figure 3b is a schematic cross-sectional view of the tubular sheath of figure 3a,
Figure 4a is another schematic perspective view of an example comprising a second lumen having a tearable wall section,
Figure 4b is a schematic cross-sectional view of the tubular sheath of figure 4a,
Figure 5 is a cross-sectional side view of the proximal ends of the tubular sheath and the elongate introducer according to some embodiments of the invention, and
Figure 6 is a schematic perspective view showing an assembly of parts according to the invention after the guidewire has been externalized from the tubular sheath.

### Detailed Description of the Invention

In the following, whenever referring to a proximal end of an element of the invention, the referral is to the end adapted for being inserted first into the patient. Whenever referring to the distal end of an element, the referral is to the end opposite the insertion end. In other words, the proximal end is the end closest to (or inside the body of) the user, when the element is to be (or is) inserted and the distal end is the opposite end - for some elements of the invention the distal end is located externally of the user's body when it is used.

Furthermore, when referring to a tapered end of an element, this may include and be seen as a frustoconical shape of the end of the element.

The longitudinal direction is the direction from the distal to the proximal end. The transverse direction is the direction perpendicular to the longitudinal direction, which corresponds to the direction across the shaft of the element.

In a first aspect, the invention relates to an assembly of parts for an endourological procedure comprising an elongate introducer and an elongate tubular sheath removably engaged onto the introducer,
- the introducer comprises at least one passageway along at least a part of its longitudinal extent defined between a tapered proximal end and a distal end,
- the tubular sheath comprises at least two lumens, wherein
- at least a first of said lumens extends between a proximal end and a distal end of the tubular sheath, and
- a second of the at least two lumens being openable for positioning of a guidewire next to the tubular sheath under the action of a peel force.

Due to the second lumen being openable, the guidewire being used for driving up the elongate tubular sheath and the elongate introducer to the correct position inside the ureter may be the only, or single, guidewire necessary when the assembly according to the invention is being used in an endourological procedure. Thereby, when the assembly has been introduced into the patient by being advanced over and along the guidewire, the tubular sheath functions as an access sheath.

The tubular sheath may comprise one or more reinforcing elements embedded in its wall along at least a part of the longitudinal extent of the sheath such as a coiled thread. This reinforced tube may thus be flexible, but resistant to collapse, compression and plication. By way of example, but not exclusively, such reinforcing element may be provided in order to avoid kinking and/or twisting of the assembly during insertion into the body of the patient.

The passageway may be a fluid channel for injecting fluids such as saline or X-ray contrasting fluid into the area of intervention before and during the procedure. The passageway may communicate with one or more fluid inlet/outlet holes at or near the proximal end of the introducer. The passageway and communicating holes may also function as a means for draining excessive fluid material and/or bodily fluids from the patient.

A sheet material is at least partly attached to the external surface of the tubular sheath and configured to define said second lumen.

The sheet material may be a plastic and/or polymer material such as, but not limited to, polyethylene (PE) or polypropylene (PP) and have a thickness appropriate for its corresponding function, i.e. solid enough to be holding a guidewire in place in the second lumen defined by the sheet material without any risk of unintentional or untimely breaking of the sheet material while also being able to be removed or torn without the use of excessive force. This will be further explained below.

In the present context, the phrase "at least partly attached to" should be understood as meaning that one or more parts, or sections, of the surface of the sheet material facing the external surface of the tubular sheath, is/are attached to said external surface. By way of example, the sheet material may be attached to the external surface in two or more rows in side-by-side relationship along at least a part of the longitudinal extent of the tubular sheath. This may be done so as to configure the sheet material to define the second lumen between such two rows of attachment.

The attachment of the sheet material to the external surface of the tubular sheath may be done by welding, such as heat welding, or by adhering or gluing the sheet material onto said surface.

The configuration of the sheet material to build the second lumen should ensure that the guidewire that is going to run inside the second lumen can do so with only insignificant or no frictional resistance encountered inside the lumen. As an example of such configuration, a slight distance between the external surface of the tubular sheath and the facing surface of the sheet material approximately halfway between the rows of attachment may be employed. That distance may preferably, but not exclusively, be equal to or up until approximately 20% larger than the diameter of the guidewire being used to ensure a satisfactory low level of frictional resistance between the guidewire and the inner surfaces of the second lumen.

In embodiments, the sheet material is tearable.

Thereby, the second lumen is openable to shift the guidewire from a guide position inside the second lumen of the tubular sheath to a security position outside and next to the tubular sheath by simply tearing apart the sheet material to "release" the guidewire from the second lumen. The sheet material may comprise at least one tear strip being a thread or thin wire e.g. embedded in the sheet material. A distal end of the tear strip may extend beyond a distal-most end of the tubular sheath. A proximal end of the tear strip embedded in the sheet material is located near, or at, the proximal-most end of the tubular sheath. By pulling the tear strip in the distal direction the sheet material starts peeling beginning at the proximal-most end of the tubular sheath and subsequently peels further on in the distal direction along the tubular sheath. Alternatively, the sheet material of the second lumen may comprise a pre-defined perforation, or otherwise weakened line along the tubular sheath.

In embodiments, a portion of the sheet material extends beyond the proximal end of the tubular sheath and being at least partly attached to an external surface of the proximal end of the introducer and is configured to define a continuing portion of the second lumen along the external surface of the proximal end of the introducer. The proximal end of the introducer should be understood as a part of the introducer extending beyond the proximal end of the tubular sheath. The proximal-most end of the introducer should be understood as that part of the proximal end of the introducer being driven the farthest into the patient. In such embodiments where the sheet material continues on to the proximal-most end of the introducer, and where it is tearable, a tear strip may also continue on to the proximal-most end of the introducer.

Thereby, the sheet material is also attached to the external surface of the proximal end part of the introducer to define a continuation of the second lumen all the way to the proximal-most end of the introducer. It should be borne in mind that the introducer is tapered towards its proximal-most end so as to have a reduced diameter at the proximal-most end. By continuing the sheet material to the proximal-most end of the introducer, the guidewire is centered in relation to the longitudinal axis of the introducer.

The sheet material may preferably, but not exclusively, be attached to the external surface of the proximal end part of the introducer similar to what has been described above with respect to attachment to the tubular sheath.

In embodiments, the sheet material is folded 180° at the proximal-most end of the introducer so as to extend back into the distal direction and the folded-back portion of the sheet material continues at least beyond a distal-most part of the tubular sheath. In other words, the sheet material is turned around to face, or even lie on top of, itself along the length of the tubular sheath and extends back in the distal direction to go past a distal-most end of the tubular sheath located outside the patient's body. By providing a distally directed pulling force to a portion of the sheet material extending beyond the distal-most end of the tubular sheath, the sheet material starts peeling beginning at the proximal-most end of the introducer and continues to peel off from the attachment(s) along the external surface of the tubular sheath all the way in the distal direction until the sheet material is completely unattached from the tubular sheath outside the patient's body. In other words, a peel force may act on the sheet material to release it from the attachment with the tubular sheath.

Thereby, the guidewire may be shifted from a guide position inside the second lumen to a security position outside, and next to, the tubular sheath. In other words, the same single guidewire first used as a working guidewire for inserting the assembly in the correct position, may now be used as the required security (or safety) wire. No use of two individual guidewires is thus required.

In a series of examples not forming part of the claimed invention, a wall section of the second lumen is tearable.

According to these examples, a section or part of the wall of the second lumen can be torn so as to open the second lumen of the tubular sheath for letting the guidewire exit the second lumen and be positioned outside and next to the tubular sheath. The wall section may comprise at least one tear strip being a thread or thin wire embedded in the wall section. A distal end of the tear strip may extend beyond the distal-most end of the tubular sheath. A proximal end of the tear strip e.g. embedded in the wall section is located near, or at, the proximal-most end of the tubular sheath.

By pulling the tear strip in the distal direction, the wall section starts peeling beginning at the proximal-most end of the tubular sheath and subsequently peels in the distal direction along the tubular sheath. Again, the guidewire may then be shifted from a guide position inside the second lumen to a security position outside, and next to, the tubular sheath, as described above for the embodiments relating to a sheet material.

Alternatively, the wall section of the second lumen may comprise a pre-defined perforation line along the tubular sheath.

Particularly, the guidewire may shift its position from inside to outside the second lumen (from the guide position to the security position) of the tubular sheath when the introducer is retracted from, or pulled out of, the tubular sheath.

In other examples, the introducer further comprises at least one security channel along at least a part of its longitudinal extent, the security channel extending between a proximal hole and a distal hole in the introducer, a wall section of the introducer extending between the holes being arranged to open under the action of a peeling force directed from inside to outside the security channel.

In these examples the guidewire moves longitudinally along the security channel of the introducer as well as along the second lumen of the tubular sheath for guiding the tubular sheath to the correct position for the procedure during insertion. The distal hole in the introducer may be located at, or near, the proximal-most end of the tubular sheath e.g. in an overlapping relationship, to thereby let the guidewire continue directly from the distal hole in the introducer into the second lumen of the tubular sheath. Once the tubular sheath is in the desired, correct location in the patient the tearable wall section of the second lumen may be torn to open the second lumen.

During the subsequent removal, or retraction, of the introducer out of the tubular sheath in the distal direction, the proximal-most end of the tubular sheath located between the introducer and the guidewire exerts a peeling or stripping action on the guidewire, which is directed from the inside to outside of the security channel thereby effectively opening the wall of the introducer between the holes and make the guidewire exit from the security channel. The removal of the introducer may also cause the guidewire to shift its position from inside to outside the second lumen. Consequently, the single guidewire is then outside and next to the tubular sheath functioning as a security wire for further work in connection with the procedure.

In embodiments, the proximal hole in the introducer is located in the proximal-most end of the introducer.

Thereby, the guidewire may be centered in relation to the longitudinal axis of the introducer which eases correct insertion into the correct location in the patient.

The assembly of parts according to the first aspect may be used according to the following exemplary method:
A method for positioning an elongate tubular sheath and a security guidewire next to the sheath in an endourological procedure comprising the steps of
   - positioning a single guidewire in a desired position,
   - engaging, driving up and setting up the elongate introducer with the elongate tubular sheath removably threaded thereonto by guiding the single guidewire through the second lumen to a position where a tapered proximal end of the elongate introducer is placed just distal of the proximal end of the single guidewire,
   - opening the second lumen of the tubular sheath by the action of a peel force in order to shift the single guidewire from a guide position inside the second lumen of the tubular sheath to a security position outside and next to the tubular sheath, and
   - retracting the elongate introducer from the elongate tubular sheath.

According to the exemplary method, only one single guidewire is necessary. The single guidewire is initially used as the working guidewire for bringing the tubular sheath controlably into the correct position in the urinary tract. When it has been confirmed that the tubular sheath is placed correctly (by X-ray imaging), the second lumen is opened to shift the guidewire from its guide position inside the second lumen to the security position outside of and next to the tubular sheath. In this manner the single guidewire is shifted from being a working guidewire to being a security guidewire. Thereby, instead of having to use two individual guidewires, according to the invention only a single guidewire is necessary and the number of necessary components for the procedure is thus reduced.

In an example, the opening of the second lumen of the tubular sheath is done by employing a distally directed pulling force to a portion of a sheet material provided at or near the distal-most end of the tubular sheath, the pulling force of the sheet material being transformed into a peel force action releasing the sheet material from attachment with the tubular sheath. The peeling action starts at the proximal-most end and continues in the distal direction along the length of the tubular sheath as the pulling force is employed until the sheet material is removed and disposable outside the patient's body.

Also disclosed is an access sheath comprising an elongate introducer and an elongate tubular sheath removably engaged onto the introducer,
- the introducer comprises at least one lumen along at least a part of its longitudinal extent defined between a tapered proximal end and a distal end thereof, and
- the tubular sheath comprises at least two lumens, wherein
- at least a first of said lumens extends between a proximal end and a distal end of the tubular sheath, and
- a second of the at least two lumens being openable for positioning of a guidewire next to the tubular sheath under the action of a peel force.

This access sheath may provide similar benefits and be employed in a similar manner as described above with regard to the assembly of parts according to the first aspect, however it may be used to obtain the same benefits in other surgical procedures than endourology. A method for positioning an access sheath and a security guidewire next to the sheath in a surgical procedure is therefore examplified.

Also disclosed is an access sheath comprising at least two lumens, wherein
- at least a first of the lumens extends between a proximal end and a distal end of the access sheath, and
- a second of the at least two lumens being openable for positioning of a guidewire next to the access sheath under the action of a peel force.

This access sheath may be located in the patient without using an introducer and while only using a single guidewire, thereby even further reducing the number of components for the procedure. By way of example, the second lumen of the tubular sheath is threaded onto the guidewire and driven up on the guidewire until it is in the desired location in the patient. Then, the second lumen can be opened to shift the guidewire to a security position outside and next to the tubular sheath. The proximal end of the access sheath can be tapering to improve and easing the guiding of the access sheath inside the patient.

This relates to an access sheath being used in procedures taking place in relatively large veins or bodily channels/canals not posing restrictions on the dimensions of the access sheath.

### Detailed Description of the Drawing

Initially, it shall be noted that the figures are schematic illustrations intended only to address the principles and functions of the invention and are not to be considered limiting to the scope of the attached claims. Furthermore, the figures and particularly the individually illustrated elements are not necessarily to scale, neither individually nor in relation to each other.

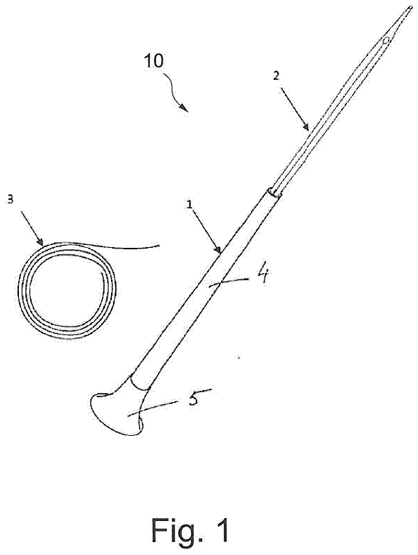

Figure 1 shows an assembly of parts 10 according to the invention comprising a tubular sheath 1 and an introducer 2 for positioning of a guidewire 3 next to the tubular sheath 1. An external surface 4 of the tubular sheath 1 is also indicated as well as a funnel or bucket 5 at a distal end of the tubular sheath 1 is shown. The bucket 5 is provided for an easy introduction of instruments and tools.

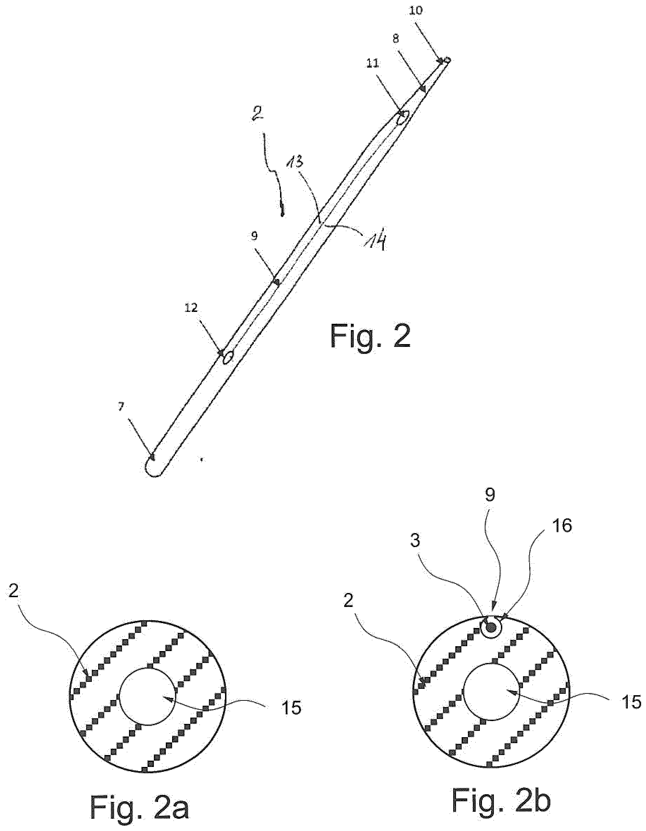

Figure 2 shows an embodiment of the introducer 2 of figure 1 in more detail. The elongate introducer 2 has a longitudinal extent defined between a tapered proximal end 8 and a distal end 7. In embodiments of the invention the introducer 2 has a security channel extending along at least a part of its longitudinal extent between a proximal 11 and a distal hole 12, the security channel comprising an openable wall section 9 between the holes that a guidewire 3 (not shown) may be released or externalized through. A proximal-most end 10 of the introducer is also indicated. Wall sides, or lips, 13,14 on each side of the wall section 9 are also indicated.
Figure 2a is a cross-sectional view of embodiments of introducer 2 without a security channel and further at least one passageway 15 of the introducer is indicated. The passageway is not necessarily limited to a centered position in the introducer.
Figure 2b is another cross-sectional view of alternative embodiments of the introducer 2 showing a security channel 16 and the passageway 15. The security channel 16 has an openable wall section 9 through which a guidewire 3 can be externalized under the action of a peeling force directed from inside to outside the security channel 16.

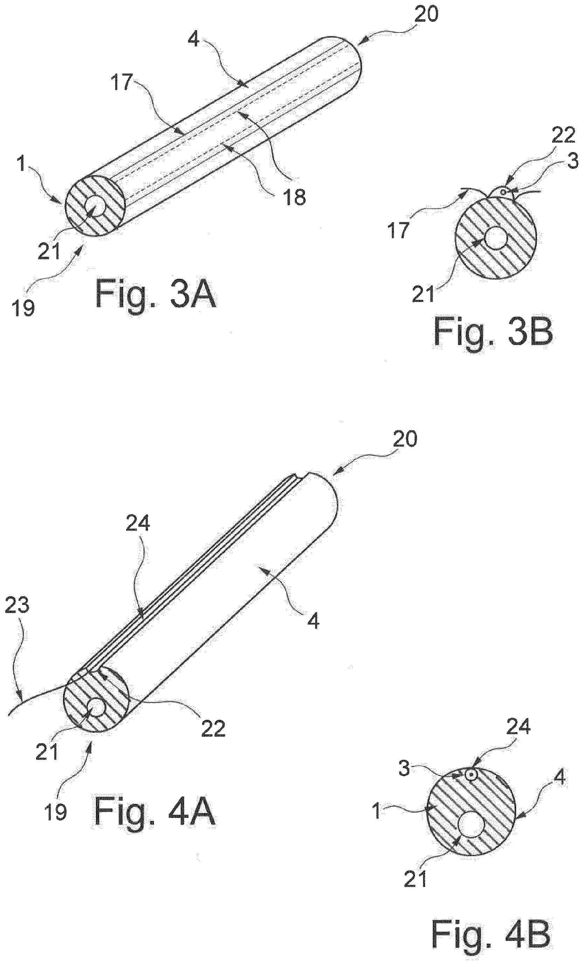

Figure 3a shows a schematic perspective view of embodiments of a tubular sheath 1 having a first lumen 21 extending between a proximal end 20 and a distal end 19. Figure 3b is a schematic cross-sectional view of the tubular sheath 1 wherein a second openable lumen 22 is defined by a sheet material 17 being attached to an external surface 4 of the tubular sheath 1. Figure 3a further indicates that the attachment is done by gluing or welding a surface of the sheet material 17 facing the external surface 4 in two side-by-side rows, indicated by 18. The attachments in the rows 18 may be interrupted or uninterrupted. Figure 3b also shows a guidewire 3 inside the not yet open second lumen 22. The size of the sheet material 17 and the distances between the sheet material 17 and the external surface 4, particularly in figure 3b, are shown exaggerated. Furthermore, the sheet material 17 may be flush with the external surface 4 to not exhibit any loose ends of material. The drawing is merely schematic to underline the presence of the sheet material 17 configured to define the second lumen 16.
Figure 4a shows a schematic perspective view of an example of a tubular sheath 1 having a first lumen 21 extending between a proximal end 20 and a distal end 19 of the sheath 1, and a second openable lumen 22 having a tearable wall section 24. In order to be able to tear open the wall section 24, a tear strip 23 is embedded in the wall section 24 all along the longitudinal extent of the tubular sheath 1 ending at the proximal end 20 of the sheath 1. Figure 4b further shows a guidewire 3 inside the not yet open second lumen 22.

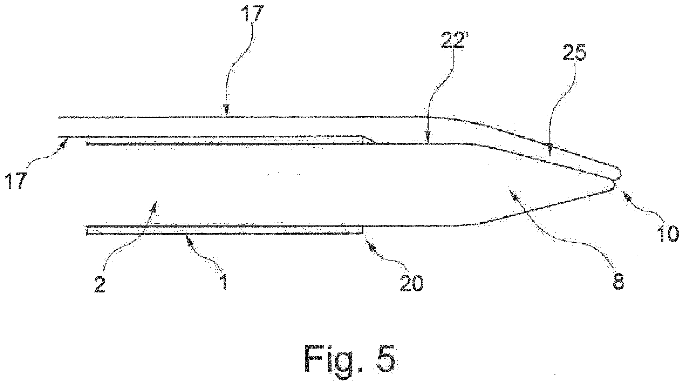

Figure 5 shows a cross-sectional side view of the proximal end 20 of the tubular sheath 1 and the proximal tapered end 8 of the introducer 2. The figure shows embodiments of the invention wherein the second lumen 22 is defined by a sheet material 17 attached to the external surface 4 of the tubular sheath 1. Figure 5 further shows embodiments wherein the sheet material 17 extends beyond the proximal end 20 of the tubular sheath 1 and is attached to an external surface 25 of the proximal end 8 of the introducer 2, thereby defining a continuing portion 22' of the second lumen 22 along the external surface 25 of the introducer. At the proximal-most end 10 of the introducer 2 the sheet material 17 is folded 180° so as to extend back in the distal direction of the tubular sheath 1. Distances between the folded layers of the sheet material 17 are shown exaggerated. The introducer may according to described embodiments also include proximal and distal holes and/or an openable security channel, however these parts are not shown in figure 5.

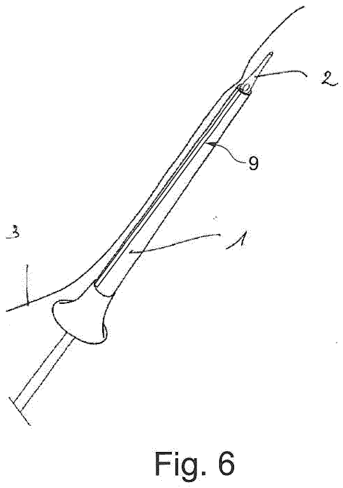

Figure 6 is a schematic perspective view showing the assembly of parts after the guidewire 3 has been externalized from the tubular sheath 1 through the openable wall section 9 (or tearable wall section 24 of the second lumen 22).

## Claims

1. An assembly of parts (10) for an endourological procedure comprising an elongate introducer (2) and an elongate tubular sheath (1) removably engaged onto the introducer (2),
- said introducer (2) comprises at least one passageway (15) along at least a part of its longitudinal extent defined between a tapered proximal end (8) and a distal end (7), and
- said tubular sheath (1) comprises at least two lumens (21, 22), wherein
- at least a first (21) of said lumens extends between a proximal end (20) and a distal end (19) of the tubular sheath (1), and
- a second (22) of said at least two lumens being openable for positioning of a guidewire (3) next to the tubular sheath (1) under the action of a peel force
- **characterized in that** a sheet material (17) is at least partly attached to the external surface (4) of the tubular sheath (1) and configured to define said second lumen (22).

2. An assembly according to claim 1, wherein said sheet material (17) is tearable.

3. An assembly according to any one of claims 1-2, wherein a portion of said sheet material (17) extends beyond the proximal end (20) of the tubular sheath (1) and being at least partly attached to an external surface (25) of the proximal end (8) of the introducer (2) and is configured to define a continuing portion (22') of said second lumen (22) along said external surface (25) of the proximal end (8) of the introducer (2).

4. An assembly according to claim 3, wherein said sheet material (17) is folded 180° at a proximal-most end (10) of the introducer (2) so as to extend back into the distal direction and continues at least beyond a distal-most part of the tubular sheath (1).

5. An assembly according to claim 1, wherein said introducer (2) further comprises at least one security channel (16) along at least a part of its longitudinal extent, said security channel (16) extending between a proximal hole (11) and a distal hole (12) in the introducer (2), a wall section (9) of the introducer (2) extending between said holes (11, 12) being arranged to open under the action of a peeling force directed from inside to outside the security channel (16).

6. An assembly according to claim 5, wherein said proximal hole (11) in the introducer (2) is located in the proximal-most end (10) of the introducer (2).

## Patentansprüche

1. Anordnung von Teilen (10) für ein endourologisches Verfahren, umfassend einen länglichen Einführer (2) und eine längliche röhrenförmige Hülse (1), die an dem Einführer (2) entfernbar in Eingriff steht,
- wobei der Einführer (2) mindestens einen Durchgang (15) entlang mindestens einem Teil seiner zwischen einem sich verjüngenden proximalen Ende (8) und einem distalen Ende (7) definierten Längserstreckung umfasst, und
- die röhrenförmige Hülse (1) mindestens zwei Lumen (21, 22) umfasst, wobei
- sich mindestens ein erstes (21) der Lumen zwischen einem proximalen Ende (20) und einem distalen Ende (19) der röhrenförmigen Hülse (1) erstreckt und
- ein zweites (22) der mindestens zwei Lumen unter der Wirkung einer Abschälkraft geöffnet werden kann, um einen Führungsdraht (3) neben der röhrenförmigen Hülse (1) zu positionieren,
- **dadurch gekennzeichnet, dass** ein Folienmaterial (17) mindestens teilweise an der Außenfläche (4) der röhrenförmigen Hülse (1) angebracht und dazu konfiguriert ist, das zweite Lumen (22) zu definieren.

2. Anordnung nach Anspruch 1, wobei das Folienmaterial (17) reißbar ist.

3. Anordnung nach einem der Ansprüche 1 - 2, wobei sich ein Abschnitt des Folienmaterials (17) über das proximale Ende (20) der röhrenförmigen Hülse (1) hinaus erstreckt und mindestens teilweise an einer Außenfläche (25) des proximalen Endes (8) des Einführers (2) angebracht und dazu konfiguriert ist, einen sich fortsetzenden Abschnitt (22') des zweiten Lumens (22) entlang der Außenfläche (25) des proximalen Endes (8) des Einführers (2) zu definieren.

4. Anordnung nach Anspruch 3, wobei das Folienmaterial (17) an einem proximalsten Ende (10) des Einführers (2) um 180° gefaltet ist, um sich in der distalen Richtung zurückzuerstrecken, und sich mindestens über einen distalsten Teil der röhrenförmigen Hülse (1) hinaus fortsetzt.

5. Anordnung nach Anspruch 1, wobei der Einführer (2) ferner mindestens einen Sicherheitskanal (16) entlang mindestens einem Teil seiner Längserstreckung umfasst, wobei sich der Sicherheitskanal (16) zwischen einem proximalen Loch (11) und einem distalen Loch (12) im Einführer (2) erstreckt, wobei ein sich zwischen den Löchern (11, 12) erstreckender Wandabschnitt (9) des Einführers (2) so angeordnet ist, dass er sich unter der Wirkung einer Abschälkraft öffnet, die von innerhalb des Sicherheitskanals (16) nach außerhalb gerichtet ist.

6. Anordnung nach Anspruch 5, wobei das proximale Loch (11) im Einführer (2) im proximalsten Ende (10) des Einführers (2) angeordnet ist.

## Revendications

1. Ensemble de pièces (10) pour une intervention endo-urologique comprenant un dispositif d'introduction (2) allongé et une gaine tubulaire (1) allongée ajustée de manière amovible sur le dispositif d'introduction (2),
- ledit dispositif d'introduction (2) comprend au moins un passage (15) le long d'au moins une partie de son étendue longitudinale définie entre une extrémité proximale (8) effilée et une extrémité distale (7), et
- ladite gaine tubulaire (1) comprend au moins deux lumières (21, 22),
- au moins une première (21) desdites lumières s'étendant entre une extrémité proximale (20) et une extrémité distale (19) de la gaine tubulaire (1), et
- une deuxième (22) desdites au moins deux lumières peut être ouverte à des fins de positionnement d'un fil-guide (3) de manière adjacente à la gaine tubulaire (1) sous l'action d'une force d'arrachement
- **caractérisé en ce qu'**un matériau en feuille (17) est au moins partiellement fixé à la surface extérieure (4) de la gaine tubulaire (1) et configuré pour définir ladite deuxième lumière (22) .

2. Ensemble selon la revendication 1, dans lequel ledit matériau en feuille (17) peut être déchiré.

3. Ensemble selon l'une quelconque des revendications 1 et 2, dans lequel une partie dudit matériau en feuille (17) s'étend au-delà de l'extrémité proximale (20) de la gaine tubulaire (1) et étant au moins partiellement fixée à une surface extérieure (25) de l'extrémité proximale (8) du dispositif d'introduction (2) et est configurée pour définir une partie de prolongement (22') de ladite deuxième lumière (22) le long de ladite surface extérieure (25) de l'extrémité proximale (8) du dispositif d'introduction (2).

4. Ensemble selon la revendication 3, dans lequel ledit matériau en feuille (17) est plié sur 180° au niveau d'une extrémité en position proximale extrême (10) du dispositif d'introduction (2) de façon à s'étendre en sens inverse dans la direction distale et continue au moins au-delà d'une partie en position distale extrême de la gaine tubulaire (1).

5. Ensemble selon la revendication 1, dans lequel ledit dispositif d'introduction (2) comprend en outre au moins un canal de sûreté (16) le long d'au moins une partie de son étendue longitudinale, ledit canal de sûreté (16) s'étendant entre un trou proximal (11) et un trou distal (12) dans le dispositif d'introduction (2), une section de paroi (9) du dispositif d'introduction (2) s'étendant entre lesdits trous (11, 12) étant conçue pour s'ouvrir sous l'action d'une force d'arrachement dirigée de l'intérieur vers l'extérieur du canal de sûreté (16).

6. Ensemble selon la revendication 5, dans lequel ledit trou proximal (11) dans le dispositif d'introduction (2) est situé dans l'extrémité en position proximale extrême (10) du dispositif d'introduction (2).
